Europäisches Patentamt

European Patent Office

Office européen des brevets

(11) Veröffentlichungsnummer: **0 173 210**
**B1**

(12) **EUROPÄISCHE PATENTSCHRIFT**

(45) Veröffentlichungstag der Patentschrift: **16.05.90**

(51) Int. Cl.⁵: **A 61 K 9/52**

(21) Anmeldenummer: **85110362.2**

(22) Anmeldetag: **19.08.85**

(54) **Pellet-Zubereitung.**

(30) Priorität: **30.08.84 DE 3431861**

(43) Veröffentlichungstag der Anmeldung:
**05.03.86 Patentblatt 86/10**

(45) Bekanntmachung des Hinweises auf die
Patenterteilung:
**16.05.90 Patentblatt 90/20**

(84) Benannte Vertragsstaaten:
**AT BE CH DE FR GB IT LI NL SE**

(56) Entgegenhaltungen:
**EP-A-0 080 341**
**US-A-3 065 142**
**US-A-4 193 985**

**Die Tablette, Dr.W.A. Ritschel, 1966, Seiten
43-48 und 77-78, 85, 93**

**Die Akte enthält technische Angaben, die nach
dem Eingang der Anmeldung eingereicht
wurden und die nicht in dieser Patentschrift
enthalten sind.**

(73) Patentinhaber: **Troponwerke GmbH & Co. KG
Berliner Strasse 156
D-5000 Köln 80 (DE)**

(72) Erfinder: **Dell, Hans-Dieter, Dr.
Kalmuentener Strasse 5
D-5060 Bergisch-Gladbach 2 (DE)**
Erfinder: **Kraus, Reinhold
Paffendorfstrasse 77
D-5000 Köln 91 (DE)**
Erfinder: **Schierstedt, Detlef, Dr.
Henri Dunantstrasse 2
D-5202 St. Augustin 3 (DE)**

(74) Vertreter: **Mann, Volker, Dr. et al
c/o Bayer AG Konzernverwaltung RP
Patentabteilung
D-5090 Leverkusen 1, Bayerwerk (DE)**

**Beschreibung**

Die Erfindung betrifft Acemetacin-Retard-Zubereitungen, Tabletten und Kapseln, die solche Zubereitungen enthalten, sowie Verfahren zur Herstellung dieser Zubereitungen.

Bei der Entwicklung eines pharmazeutischen Retard-Präparates wird das Ziel verfolgt, Blutspiegelspitzenwerte zu reduzieren und die Zeit, in der die Wirkstoffkonzentration sich im therapeutischen Bereich befindet, zu verlängern. Eine solche Darreichungsform hat den Vorteil, daß Nebenwirkungen aufgrund erhöhter Blutspiegelspitzenwerte vermindert werden und sich die Einnahmeintervalle verlängern.

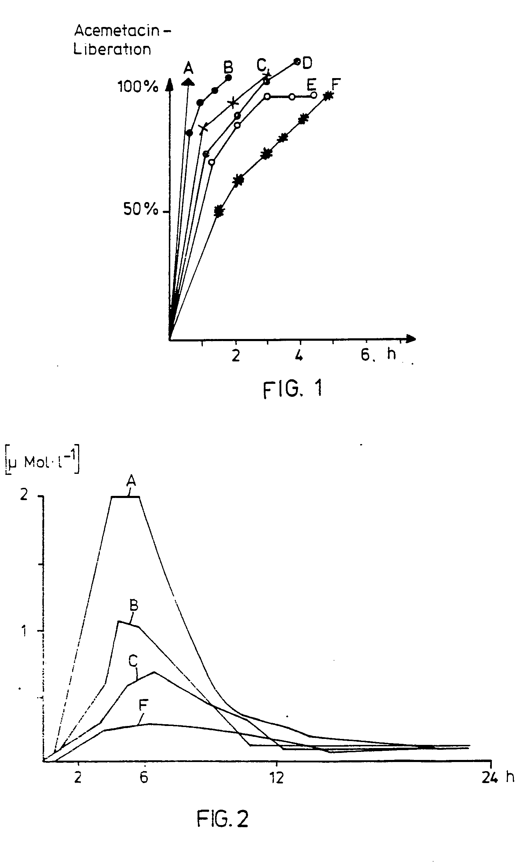

Für die Anwendung herkömmlicher Retardierungsmethoden ist nicht jeder Arzneistoff in gleichem Maße geeignet. Wird z.B. ein Arzneistoff hauptsächlich im Magen und in den vorderen Darmabschnitten (R. Gröning, Pharm. *Ind. 46*, S. 89, 1984) resorbiert ("begrenzte Resorption"), versagen herkömmliche Retardierungsmethoden. Eine in-vitro-Retardierung macht sich in einem solchen Fall in vivo nur durch eine Verminderung der Bioverfügbarkeit bemerkbar (siehe Abb. 1 und 2).

Eine solche Substanz wäre nur zu retardieren, wenn man die Verweildauer im Gastrointestinaltrakt, insbesondere im Magen, verlängert. Dies kann beispielsweise durch Erhöhung der Dichte geschehen. In der US-PS 4 193 985 ist beispielsweise eine Zubereitung beschrieben, die aus Pellets mit erhöhter Dichte und einer unlöslichen Diffusionsmembran besteht. Ein solches System ist in seinem Freisetzungsprofil hauptsächlich auf den Dünndarm ausgelegt. Da die Verweilzeiten im Dünndarm durch viele Faktoren variieren, kann ein solches Prinzip für Arzneistoffe, die nur in relativ kurzen Magen-Darm-Abschnitten resorbiert werden, nicht angewendet werden.

Die EP—A—00 80 341 beschreibt eine Retardformulierung für den Wirkstoff Indometacin auf Basis von Mehrfacheinheitsdosen, wobei die den Wirkstoff und Formulierungshilfsmittel enthaltenden Kerne mit einem magensaftresistenten Lack überzogen sind. Der Wirkstoff Indometacin unterscheidet sich jedoch wesentlich in seinen Eigenschaften von Acemetacin. Die Maßnahmen gemäß EP—A—00 80 341 führen bei Acemetacin nicht zum Erfolg.

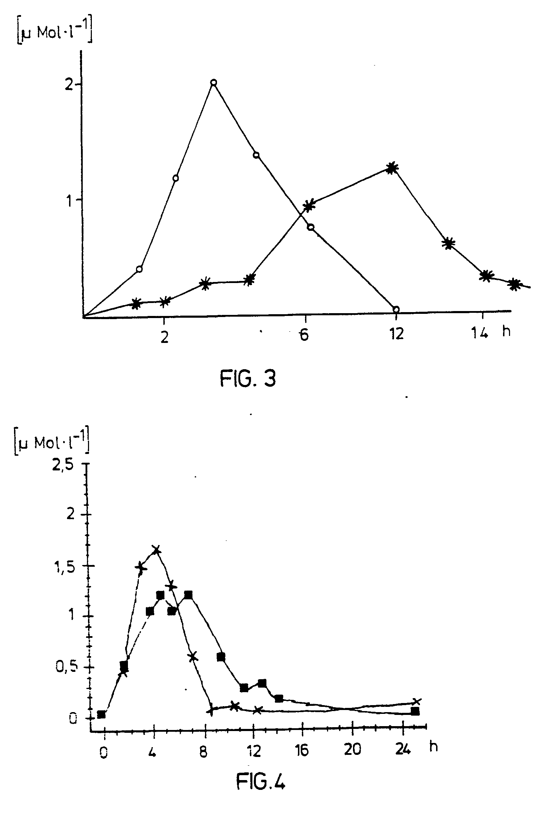

Überraschenderweise wurde gefunden, daß die erfindungsgemäßen Zubereitungen für eine Retardierung von Acemetacin mit begrenzter Resorption hervorragend geeignet sind. [Ein in-vivo-Vergleich der erfindungsgmemäßen Acemetacin-Retard-Formulierung mit unretardiertem Acemetacin an zwei gesunden Probanden zeigt eine Verschiebung des Blutspiegelmaximums von ca. 4 auf ca. 8 Stunden ohne Verminderung der relativen Bioverfügbarkeit (s. Abb. 3).]

Gegenstand der Erfindung ist daher eine Retard-Zubereitung von Acemetacin auf Basis von eingekapselten Mehrfacheinheitsdosen in Form von Pellets, die eine physiologisch unschädliche Substanz mit höherem spezifischen Gewicht enthalten, dadurch gekennzeichnet, daß sie aus einer Initialdosis, die 10 bis 60 Gew.-% der Gesamtmenge des Acemetacins und gegebenenfalls Zuschlagstoffe enthält, und

einer Pellet-Zubereitung, die die Restmenge des Wirkstoffs, ein Sprengmittel, ein Bindemittel und eine Substanz mit höherem spezifischen Gewicht enthält und mit einem magensaftresistenten Lack überzogen ist, wobei die einzelnen Pellets einen Durchmesser von 0,2 bis 1,8 mm aufweisen, besteht.

Bevorzugt ist die scheinbare Dichte 1,5 bis 1,8, besonders bevorzugt 1,6.

Die Pellet-Zubereitungen können 0,1 bis 80 Gew.-% Acemetacin enthalten. Das Beschwerungsmittel liegt in Konzentration von 20 bis 98 Gew.-% vor und der Rest (ad 100 Gew.-%) besteht aus Bindemittel und weiteren Zuschlagstoffen wie Sprengmitteln etc. Die Mengenangaben beziehen sich auf das unlackierte Pellet.

Die Dichte der Pellet-Zubereitung kann durch spezifisch schwere Stoffe wie $TiO_2$, $BaSO_4$, Ferrum reductum und Eisenoxide eingestellt werden. Die Beschwerungsmittel werden bevorzugt in Mengen von 40 bis 70 Gew.-%, bezogen auf das unlackierte Pellet, eingesetzt.

Die Größe (Durchmesser) der erfindungsgemäßen Pellets liegt bei 0,2 bis 1,8 mm, bevorzugt bei 0,8 bis 1,5 mm, besonders bevorzugt 0,5 bis 1,25 mm.

Die erfindungsgemäßen Acemetacin-Retard-Zubereitungen, bestehend aus Pellet-Zubereitung und Initialdosis, erfüllen die eingangs gewünschten Eigenschaften voll.

Die Acemetacin-Retard-Zubereitungen lassen sich zu Tabletten verpressen oder in Kapseln abfüllen. Besonders bevorzugt beträgt die Initialdosis, die auch als Pulver, gegebenenfalls mit anderen Zuschlagstoffen, aber auch in anderer als Pulverform zugefügt wird, 1/3 der Gesamtmenge an Acemetacin.

Bevorzugt für die erfindungsgemäßen Acemetacin-Retard-Zubereitungen sind 60 bis 200 mg Acemetacin.

Für die Lackierung der Pellets kann im Prinzip jeder Lack Verwendung finden, der zu magensaftresistenten Filmüberzügen führt, z.B. Celluloseacetat-Phthalat, Hydroxypropylmethylcellulose-Phthalat und andere Cellulose-Phthalat-Derivate, Polymerisate von Methacrylsäure und Methacrylsäureestern (Eudragit® L und S), Methacrylsäure-Acrylsäuremethylester-kopolymerisat (Eudragit®L 30 D).

Die Bestimmung der scheinbaren Dichte der Pellet-Zubereitungen kann pygnometrisch mit einem Suspensionsmittel, in dem der Lacküberzug unlöslich ist, erfolgen.

Die Herstellung der Pellet-Zubereitungen erfolgt durch Mischen des Wirkstoffs mit dem Beschwerungsmittels und dem Bindemittel und anschließendes Pelletieren oder Granulieren.

Die Granulierung erfolgt beispielsweise nach den Methoden, die in "Ullmann, Bd. 18, Pharmazeutische Technologie" angegeben sind.

Feuchtgranulierung

Bei der Feuchtgranulierung wird die Pulvermischung mit einer Flüssigkeit zu einem Teig verarbeitet, aus dem nach Trocknung, Verdunsten oder Erstarren der Granulationsflüssigkeit das Granulat entsteht. Man unterscheidet:

a) Aufbauende Granulierung.

Das in Bewegung gehaltene Pulvergemisch wird durch Besprühen mit Granulierflüssigkeit auf die gewünschte Granulatgröße gebracht.

Beim Tellergranulierverfahren wird das zu granulierende Pulvergemisch auf einen schräg rotierenden Teller gebracht. Das entstehende Granulat ist kugelförmig. Bei der Wirbelschicht-Granulierung wird das Pulvergemisch in einem Luftstrom aufgewirbelt, in Schwebe gehalten und mit der Granulationsflüssigkeit besprüht. Auch hier entstehen weitgehend kugelförmige Granulatkörner.

b) Abbauende Granulierung.

Das Pulvergemisch wird mit der Granulierflüssigkeit durch Ankneten mit einem Kneter gleichmäßig durchfeuchtet und in Teigkonsistenz durch ein Sieb oder eine Lochwalze bzw. Lochscheibe gepreßt. Nach dem Trocknen wird das zylindrische oder quaderförmige Granulat gebenenfalls auf die gewünschte Korngröße ausgesiebt.

Als Granulierflüssigkeit verwendet man je nach der Löslichkeit des Wirkstoffes vorzugsweise Wasser, daneben z.B. Wasser-Alkohol-Gemische oder Lösungen von Bindemitteln. Geeignete Bindemittel sind makromolekulare Stoffe wie Cellulose-Derivate, Gelatine, Stärke, Polyvinylpyrrolidon oder Alginate.

Schmelzgranulieren

Durch Schmelzen von Bestandteilen der Pulvermischung und ihre Verformung durch Siebe entstehen Sinter- oder Schmelzgranulate.

Trockengranulierung

Bei der Trockengranulierung wird das Pulvergemisch in Tablettenmaschinen oder Walzen zu relativ großen, groben Preßlingen, sog. Briketts geformt, grob zerkleinert und auf die gewünschte Granulatgröße gesiebt. Man wendet das Verfahren besonders bei Verarbeitung thermolabiler oder feuchtigkeitsempfindlicher Wirkstoffe an.

Zur Herstellung von erfindungsgemäßen Tabletten wird auf übliche Tablettierungsverfahren zurückgegriffen (s. z.B. Ullmann, Bd. 18, Pharmazeutische Technologie).

Bei der Tablettierung wird die Pellet-Zubereitung bzw. Granulat-Zubereitung in Gegenwart von freiem Wirkstoff (Initialdosis) und anderen Zuschlägen durch Anwendung von hohem Druck in ein Komprimat überführt. Durch Kombination mit entsprechenden Hilfsstoffen kann fast jeder Wirkstoff in eine Tablette überführt werden, die zudem noch bestimmte, für die Anwendung erforderliche Eigenschaften haben kann.

Über die galenischen Eigenschaften von Tabletten, insbesondere ihren Zerfall in Wasser oder in den Verdauungssäften und ihre mechanischen Charakteristika entscheiden neben dem Herstellungsverfahren die Menge und die physikalisch-chemischen Eigenschaften der Wirk- und Hilfsstoffe.

*Hilfsstoffe* für die Tablettenherstellung werden in folgende wichtigste Typen eingeteilt:

Füllstoffe, z.B. Milchzucker, Rohrzucker

Bindemittel, z.B. Stärke, Gelatine, Zucker, Cellulose-ether, Polymere, z.B. Polyvinylpyrrolidon

Sprengmittel, z.B. Stärke, Stärkeether, Kollidon® CL

Gleit- und Formentrennmittel, z.B. Talkum, Stearate, Silicone

Fließreguliermittel, z.B. hochdisperses Siliciumdioxid, Talkum.

Die Kapsel-Herstellung mit erfindungsgemäßen Acemetacin-Retard-Zubereitungen kann ebenfalls nach **Methoden** erfolgen, die in "Ullmann, Bd. 18, Pharmazeutische Technologie" beschrieben sind, erfolgen.

Durch Variation des Bindemittels, der Bindemittelkonzentration in den Pellets bzw. Granulaten und des Herstellungsverfahrens ist es möglich, die Auflösungszeit des pharmazeutischen Wirkstoffs im Dünndarm zu steuern.

Als Sprengmittel können z.B. Kollidon® CL, Stärke, UAP, Avicel® etc., oder ein Zerfallsbeschleuniger, wie Aerosil®, Tenside etc. eingesetzt werden.

(Kollidon®CL=vernetztes Polyvinylpyrrolidon

UAP=Ultraamylopektin

Avicel®=mikrokrist. Cellulose

Aerosil®=hochdisperses $SiO_2$)

Die magensaftresistente Lackierung der Pellet-Zubereitungen kann nach den üblichen Lackierungsverfahren, wie z.B. Wirbelschicht und offene oder geschlossene Kesselsystem, erfolgen.

Beispiele

Die Initialdosis kann in Form einer Pulvermischung oder als Tablette zusammen mit den magensaftresistenten Pellets in Kapseln abgefüllt werden (z.B. 10 bis 60% Initialdosis, bevorzugt 1/3 der Gesamtdosis).

In den Abbildungen ist auf der x-Achse die Zeit t in Stunden aufgetragen.

In den Abbildungen 2, 3 und 4 ist auf der y-Achse der Blutspiegelwert in µmol/l angegeben.

Abb. 1 zeigt die "in-vitro"-Freisetzung von Acemetacin aus Acemetacin-Diffusionspelletsformulierungen (B, C, F) und unretardiertem Acemetacin (A). USP Paddle/900 ml künstlicher Darmsaft 100U/min.

Abb. 2 zeigt einen Blutspiegelvergleich von Acemetacin und Acemetacin-Diffusionspelletformulierungen. Die Kurve A betrifft ein Acemetacin-Handelspräparat. Die Kurven B, C und F beschreiben Retardierungsversuche nach dem Stand der Technik.

Dosis: jeweils 120 mg Acemetacin

Abb. 3 zeigt einen Blutspiegelvergleich an zwei Probanden. [Ein in-vivo-Vergleich der erfindungsgemäßen Acemetacin-Retard-Formulierung mit unretardiertem Acemetacin an den zwei gesunden Probanden zeigt eine Verschiebung des Blutspiegelmaximums von ca. 4 auf ca. 8 Stunden ohne Verminderung der relativen Bioverfügbarkeit (s. Abb. 3).]

Abb. 4 zeigt den Blutspiegelverlauf nach Applikation von Acemetacin:
Kästen=Retardformulierung erfindungsgemäß
Kreuze=Normalform (nicht redardiert)
Mittelwerte (n=4);
appliziert=90 mg Wirkstoff

Rezepturen von erfindungsgemäßen Retard-Zubereitungen

|  | Acemetacin | |
| --- | --- | --- |
| Wirkstoff | 60 | 60 |
| Titandioxid | 75,6 | 94 |
| Kollidon® CL | 7,8 | |
| Kollidon® 25 | 14,4 | 13,6 |
| Celluloseacetat-Phthalat | 20,5 | 20,5 |
| Talcum | 1,0 | 1,0 |
| Triacetin | 4,6 | 4,6 |

In-vivo-Untersuchungen

Der Retardeffekt konnte in-vivo durch Humanstudien, welche im Vergleich zu nicht retardiertem Wirkstoff Acemetacin in Form des Handelsproduktes Rantudil®, bewiesen werden.

In Abb. 4 ist in Form von Mittelwertkurven der unterschiedliche Verlauf der Blutspiegelkurven zu erkennen (4 Probanden).

An einer größeren Probandenzahl wurden im randomisierten cross-over-Versuch die für Retardformulierungen wichtigen (Merkmale) (nach J. Meier, E. Nülsch und R. Schmidt, Eur. J. Clin. Pharmacol. 7, 429, 1974, P. Guissou, G. Guisinaud, G. Llorca, E. Lejeune und J. Sasard, Eur. J. Clin. Pharmacol. 24, 667, 1983, R. Verbesselt. T. B. Tjandramaga, A. Mullier, P. J. De Schepper, T. Cook, C. Derouwaux, R. Kramp und S. Hwang, Eur. J. Clin. Pharmacol. 24, 563, 1983) vergleichend untersucht.

Hinsichtlich der Zeit des maximalen Blutspiegels ($t_{max}$), der maximalen Blutspiegelkonzentration ($C_{max}$), der Blutspiegelhöhe (µMol/l) zu verschiedenen Zeiten post applicationem, der Absorptionshalbwertszeit, der Halbwertsdauer und der Retardquotient erwies sich die Retardformulierung als echtes Retardpräparat. Die Unterschiede zum nicht retardierten Präparat sind statistisch gesichert.

Beispiel für die Herstellung von Pellet-Zubereitungen für Hartgelatinekapseln
Für das unlackierte Granulat werden folgenden Komponenten eingesetzt:

| I | Acematacin | 60,0 mg |
| --- | --- | --- |
|  | Titandioxid | 75,6 mg |
|  | Kollidon® CL | 7,8 mg |
| II | Kollidon® 25 (Lösung) | 14,4 mg |

Der magensaftresistente Lack besteht aus folgenden Bestandteilen:

| III | Celluloseacetatphthalat | 20,5 mg |
| --- | --- | --- |
|  | Triacetin | 4,6 mg |
|  | Talkum | 1,0 mg |

Komponenten I werden in einem Schnellmischer 2 Minuten gemsicht (286 Upm). Anschließend wird die Lösung II in einem Guß bei laufendem Mischer (ca. 2 Minuten) hinzugegeben.

Die Feuchtmasse wird mit einem Lochscheiben-Granulator (1,5 mm Bohrung) granuliert und bei 50°C getrocknet. Anschließend wird durch ein 1 mm Sieb verkleinert und die Staubanteile abgetrennt.

Die Lackierung erfolgt in einem Wirbelschicht-Granulator. Die Komponenten III werden in 10% Aceton-Lösung (bezogen auf das Celluloseacetatphthalat in der Wirbelschicht) auf das Granulat aufgesprüht. Man erhält hier gleichmäßige Pellet des Wirkstoffs.

Die Pellets werden anschließen in die Hartgelatinekapseln gefüllt.

## Patentansprüche

1. Retard-Zubereitung von Acemetacin auf Basis von eingekapselten Mehrfacheinheitsdosen in Form von Pellets, die eine physiologisch unschädliche Substanz mit höherem spezifischen Gewicht enthalten, dadurch gekennzeichnet, daß sie aus

einer Initialdosis, die 10 bis 60 Gew.-% der Gesamtmenge des Acemetacins und gegebenenfalls Zuschlagstoffe enthält, und

einer Pellet-Zubereitung, die die Restmenge des Wirkstoffs, eine Sprengmittel, ein Bindemittel und eine Substanz mit höherem spezifischen Gewicht enthält und mit einem magensaft-resistenten Lack überzogen ist, wobei die einzelnen Pellets einen Durchmesser von 0,2 bis 1,8 mm aufweisen, besteht.

2. Retard-Zubereitung nach Anspruch 1, dadurch gekennzeichnet, daß die Initialdosis als Pulver oder als Tablette vorliegt.

3. Retard-Zubereitung nach den Ansprüchen 1 und 2, dadurch gekennzeichnet, daß man als Sprengmittel Stärke, Stärkeether, Ultraamylopectin, mirkokristalline Cellulose oder vernetztes Polyvinylpyrrolidon einsetzt.

4. Retard-Zubereitung nach den Ansprüchen 1 bis 3, dadurch gekennzeichnet, daß man als Bindemittel Stärke, Gelatine, Zucker, Celluloseether oder Polyvinylpyrrolidon einsetzt.

5. Retard-Zubereitung nach den Ansprüchen 1 bis 4, dadurch gekennzeichnet, daß sie als physiologisch unschädliche Substanz Titandioxid, Bariumsulfat, Ferrum reductum oder Eisenoxide enthalten.

6. Retard-Zubereitung nach den Ansprüchen 1 bis 5, dadurch gekennzeichnet, daß sie 40 bis 70 Gew.-% der physiologisch unschädlichen Substanz, bezogen auf das unlackierte Pellet, enthalten.

7. Retard-Zubereitung nach den Ansprüchen 1 bis 6, dadurch gekennzeichnet, daß man als magensaft-resistenten Lack Cellulose-acetal-Phthalat, Hydroxypropylmethyl-cellulose Phthalat oder dessen Derivate, Polymerisate von (Meth)-acrylsäure oder (Meth)-acrylsäureester oder Copolymerisate aus (Meth)-acrylsäure und Acrylsäuremethylester verwendet.

8. Retard-Zubereitung nach den Ansprüchen 1 bis 7, dadurch gekennzeichnet, daß sie 60 bis 200 mg Acemetacin enthält.

9. Verfahren zur Herstellung von Retard-Zubereitungen von Acemetacin bestehend aus

einer Initialdosis, die 10 bis 60 Gew.-% der Gesamtmenge des Acemetacins und gegebenenfalls Zuschlagstoffe enthält, und

einer Pellet-Zubereitung, die die Restmenge des Wirkstoffs, ein Sprengmittel, ein Bindemittel und eine Substanz mit höherem spezifischen Gewicht enthält und mit einem magensaft-resistenten Lack überzogen ist, wobei die einelnen Pellets einen Durchmesser von 0,2 bis 1,8 mm aufweisen,

dadurch gekennzeichnet, daß man für die Initialdosis 10 bis 60% des zur Anwendung kommenden Acemetacins gegebenenfalls in Gegenwart von Zuschlagstoffen pulverisiert oder zu einer Tablette preßt, und für die Pellet-Zubereitung die Restmenge des Wirkstoffs, ein Sprengmittel, ein Bindemittel und eine Substanz mit höherem spezifischen Gewicht mischt, pelletiert, die Pellets mit einem Durchmesser von 0,2 bis 1,8 mm klassiert und diese mit einem magensaftresistenten Lack überzieht,

und dann die Initialdosis und die Pellet-Zubereitung durch eine Kapsel einschließt.

10. Verfahren nach Anspruch 9, dadurch gekennzeichnet, daß man 40 bis 70 Gew.-% der Substanz mit höherem spezifischen Gewicht einsetzt.

## Revendications

1. Préparation d'acémétacine à action retardée à base de doses unitaires multiples encapsulées sous forme de pellets, qui contiennent une substance sans danger du point de vue physiologique, de masse volumique élevée, caractérisée en ce qu'elle est constituée par une dose initiale qui contient 10 à 60% en poids de la quantité totale d'acémétacine et, le cas échéant, des additifs, et

d'une préparation en pellets, qui contient la quantité restante de substance active, un agent de désintégration, un liant et une substance de masse volumique élevée, et qui est révêtue d'une laque résistant au suc gastrique, les pellets individuels présentant un diamètre de 0,2 à 1,8 mm.

2. Préparation à action retardée suivant la revendication 1, caractérisée en ce que la dose initiale se présente sous forme de poudre ou comprimés.

3. Préparation à action retardée suivant les revendications 1 et 2, caractérisée en ce qu'on utilise comme agent de désintégration l'amidon, un éther d'amidon, l'ultra-amylopectine, de la cellulose microcristalline ou de la polyvinylpyrrolidone réticulée.

4. Préparation à action retardée suivant les revendications 1 à 3, caractérisée en ce qu'on utilise comme liant de l'amidon, de la gélatine, des sucres, des éthers de cellulose ou de la polyvinylpyrrolidone.

5. Préparation à action retardée suivant les revendications 1 à 4, caractérisée en ce qu'elle contient comme substances sans danger du point de vue physiologiqué du dioxyde de titane, du sulfate de baryum, du fer réduit ou des oxydes de fer.

6. Préparation à action retardée suivant les revendications 1 à 5, caractérisée en ce qu'elle contient 40 à 70% en poids de substance sans danger du point de vue physiologique, par rapport au pellet non revêtu de laque.

7. Préparation à action retardée suivant les revendications 1 à 6, caractérisée en ce qu'on utilise comme laque résistant au suc gastrique de l'acétatephthalate de cellulose, du phthalate d'hydroxypropylméthylcellulose ou ses dérivés, des polymérisats d'acide (méth)acrylique ou d'ester d'acide (méth)acrylique ou des copolymérisats d'acide (méth)acrylique et d'ester méthylique d'acide acrylique.

8. Préparation à action retardée suivant les revendications 1 à 7, caractérisée en ce qu'elle contient 60 à 200 mg d'acémétacine.

9. Procédé de production de préparations à action retardée d'acémétacine, constituées d'une dose initiale qui contient 10 à 60% en poids de la quantité totale d'acémétacine et, le cas échéant, d'additifs, et

d'une préparation en pellets, qui contient la quantité restante de substance active, un agent de désintégration, un liant et une substance de masse volumique élevée et qui est revêtue d'une laque résistant au suc gastrique, les pellets individuels présentant un diamètre de 0,2 à 1,8 mm,

caractérisé en ce qu'on pulvérise ou on presse en un comprimé, pour la dose initiale, 10 à 60% de l'acémétacine utilisée, le cas échéant en présence d'additifs, et pour la préparation de pellets, on mélange la quantité restante de substance active, un agent de désintégration, un liant et une substance de masse volumique élevée, on transforme le mélange en pellets, on classe les pellets d'un diamètre de 0,2 à 1,8 mm et on les revêt d'une laque résistant au suc gastrique,

puis on enferme dans une capsule la dose initiale et la préparation en pellets.

10. Procédé suivant la revendication 9, caractérisé en ce qu'on utilise 40 à 70% en poids de la substance de masse volumique élevée.

## Claims

1. Sustained-release formulation of acemetacin based on encapsulated multiple unit doses in the form of pellets containing a physiologpally innocuous substance of relatively high specific gravity, characterized in that it consists of an initial dose which contains 10 to 60% by weight of the total amount of acemetacin and if appropriate additives, and a pellet formulation which contains the remaining amount of the active compound, a disintegrating agent, a binder and a substance having a relatively high specific gravity and is coated with a lacquer which is resistant to gastric juice, the individual pellets having a diameter of 0.2 to 1.8 mm.

2. Substained release formulation according to Claim 1, characterized in that the initial dose is in the form of a powder or tablet.

3. Sustained release formulation according to Claims 1 and 2, characterized in that starch, starch ether, ultraamylopectin, microcrystalline cellulose or cross-linked polyvinylpyrrolidone is employed as the disintegrating agent.

4. Sustained-release formulation according to Claims 1 to 3, characterized in that starch, gelatin, sugar, cellulose ether or polyvinylpyrrolidon is employed as the binder.

5. Sustained-release formulation according to Claims 1 to 4, characterized in that it contains titanium dioxide, barium sulphate, ferrum reductum or iron oxides as the physiologically innocuous substance.

6. Sustained-release formulation according to Claims 1 to 5, characterized in that it contains 40 to 70% by weight of the physiologically innocuous substance, based on the non-lacquered pellet.

7. Sustained-release formulation according to Claims 1 to 6, characterized in that cellulose acetate phthalate, hydroxypropylmethyl-cellulose phthalate or derivatives thereof, polymers of (meth)acrylic acid or (meth) acrylic acid esters or copolymers of (meth)acrylic acid and methyl acrylate are used as the lacquer which is resistant to gastric juice.

8. Sustained-release formulation according to Claims 1 to 7, characterized in that it contains 60 to 200 mg of acemetacin.

9. Process for the preparation of sustained-release formulations of acemetacin consisting of an initial dose which contains 10 to 60% by weight of the total amount of acemetacin and if appropriate additives and a pellet formulation which contains the remaining amount of the active compound, a disintegrating agent, a binder and a substance of relatively high specific gravity and is coated with a lacquer which is resistant to gastric juice, the individual pellets having a diameter of 0.2 to 1.8 mm, characterized in that for the initial dose, 10 to 60% of the acemetacin used is powdered or pressed to a tablet, if appropriate in the presence of additives, and for the pellet formulation, the remaining amount of the active compound, a disintegrating agent, a binder and a substance of relatively high specific gravity are mixed, the mixture is pelletized, the pellets are classified into those having a diameter of 0.2 to 1.8 mm and these are coated with a lacquer which is resistant to gastric juice, and the initial dose and the pellet formulation are then enclosed by a capsule.

10. Process according to Claim 9, characterized in that 40 to 70% by weight of the substance of relatively high specific gravity is employed.

Acemetacin-
Liberation

FIG. 1

FIG. 2

1

FIG. 3

FIG.4

2